# EUROPÄISCHE PATENTANMELDUNG

(11) **EP 4 501 894 A1**
(43) Veröffentlichungstag der Anmeldung: **05.02.2025**
(21) Anmeldenummer: 23188953.6
(22) Anmeldetag: 01.08.2023
(51) Int. Cl.: C07C 29/151, C07C 31/04, C07C 29/78, C07C 29/80, C25B 1/00

(54) **VERFAHREN ZUR HERSTELLUNG VON METHANOL UNTER NUTZUNG DER ABWÄRME EINES ELEKTROLYSEURS**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: König, Sebastian, 60439 Frankfurt am Main (DE); Peña, Vincent, 60439 Frankfurt am Main (DE)
(74) Vertreter: Air Liquide

(57) **Zusammenfassung**

Der Erfindung betrifft ein Verfahren zur Herstellung von Methanol auf Basis eines Synthesegasstroms, welcher eine Wasserstoffkomponente und eine Kohlenstoffoxidkomponente umfasst, wobei zumindest eine der Komponenten zumindest teilweise in einem Elektrolyseur erzeugt wird. Der Synthesegasstrom wird an einem Methanolsynthesekatalysator in einer Reaktoranordnung zu einem Rohmethanolstrom umgesetzt. Methanol wird durch Destillation in einer Destillationsvorrichtung thermisch aus dem Rohmethanolstrom abgetrennt, wobei ein methanolhaltiger Strom in einem Destillationsverdampfer der Destillationsvorrichtung verdampft wird. Erfindungsgemäß ist vorgesehen, dass in dem Elektrolyseur erzeugte Abwärme durch indirekte Wärmeübertragung auf ein zirkulierendes Wärmepumpenmedium eines Wärmepumpensystems übertragen wird, wodurch das Wärmepumpenmedium zumindest teilweise verdampft, das Wärmepumpenmedium anschließend verdichtet wird, und das Wärmepumpendmedium durch zumindest teilweises Kondensieren abgegebene Wärme durch indirekte Wärmeübertragung auf ein flüssiges Medium überträgt, und die von dem Wärmepumpensystem auf das flüssige Medium übertragene Wärme zumindest teilweise für die Verdampfung des methanolhaltigen Stroms im Destillationsverdampfer genutzt wird.

## Beschreibung

### Gebiet der Erfindung

Die Erfindung betrifft ein Verfahren zur Herstellung von Methanol aus einem Synthesegasstrom, wobei der Synthesegasstrom eine Wasserstoffkomponente und eine Kohlenoxidkomponente umfasst. Das Verfahren zeichnet sich dadurch aus, dass zumindest eine der Komponenten zumindest teilweise durch einen Elektrolyseur bereitgestellt wird, und in dem Elektrolyseur erzeugte Abwärme durch ein Wärmepumpensystem für die thermische Abtrennung von Methanol aus dem in der Methanolsynthese erzeugten Rohmethanol durch Destillation genutzt wird.

### Stand der Technik

Produktionsanlagen für die Herstellung von Methanol umfassen einen Destillationsbereich zur thermischen Trennung des bei der Methanolsynthese entstehenden Rohmethanols in Methanol, Wasser und Nebenprodukte. Dafür wird Wärme auf einem Temperaturniveau von mehr als 90 °C, vorzugsweise von mehr als 120 °C, oder von mehr als 150 °C benötigt.

Ein Teil dieser Wärme kann in Form von Mitteldruckdampf bereitgestellt werden, welcher in wassergekühlten Methanol-Reaktoren auf der Mantelseite bei der Kühlung des Katalysatorbetts durch Kesselspeisewasser erzeugt wird. Dieser Mitteldruckdampf kann außerhalb des Reaktors auf einen Sattdampfdruck, welcher der zur Destillation benötigten Temperatur entspricht, entspannt und gesättigt werden. Dieser Dampf deckt jedoch nur einen Teil des Wärmebedarfs der Destillation ab, der restliche Wärmbedarf muss auf andere Weise zur Verfügung gestellt werden.

Konventionelle, auf fossilen Einsatzstoffen (zum Beispiel Erdgas) basierende Methanolanlagen nutzen Hochtemperaturwärme von häufig mehr als 300 °C aus der Synthesegaserzeugung, die in Form von Dampf verwertet wird, um die fehlende Wärme für die Methanoldestillation bereitzustellen. Beispiele für die Synthesegaserzeugung aus fossilen Einsatzstoffen sind Dampfreformierung von Methan (SMR), autotherme Reformierung (ATR), partielle Oxidation (POx), sowie katalytische partielle Oxidation (CPO). Der in der Synthesegaserzeugung gebildete Dampf kann ebenfalls auf einen Sattdampfdruck, welcher der zur Destillation benötigten Temperatur entspricht, entspannt und gesättigt werden.

Andere Verhältnisse herrschen bei Methanolproduktionsprozessen, welche nicht auf der Verwendung fossiler Einsatzstoffe für die Synthesegaserzeugung beruhen. Beispielsweise bei der sogenannten kohlendioxidbasierten Methanolsynthese wird ein Kohlendioxidstrom beispielsweise aus einer Kohlendioxidabscheideanlage vorzugsweise mit elektrolytisch erzeugtem Wasserstoff umgesetzt. Die Synthesegas-"Erzeugung" ist in diesem Fall somit lediglich die Zusammenführung der vorgenannten Ströme. Abwärme auf hohem Temperaturniveau, welche als Dampf genutzt werden kann, fällt somit nicht oder nur in geringem Ausmaß an. Da das so erzeugtes Synthesegas ferner kaum oder kein Kohlenmonoxid enthält, läuft die eigentliche Methanolbildungsreaktion mit verminderter Bildung von Wärme (Exothermie) ab, als dies bei der Nutzung von hochkohlenmonoxidhaltigem Synthesegas der Fall wäre. Somit wird auf der Mantelseite des betreffenden wassergekühlten Reaktors weniger Dampf erzeugt, als dies bei konventionellen Methanolsynthesen der Fall wäre.

Vorgenannte Prozesse weisen somit von vorneherein einen Mangel an nutzbarer Wärme auf.

Infolgedessen muss Wärme von einer außerhalb der betreffenden Anlage stammenden Quelle importiert werden. Dies geschieht meist in Form von Dampf, der aus einem nahe gelegenen Versorgungsnetz, einem elektrischen Dampfkessel, oder aus der Wärmerückgewinnung innerhalb der den Kohlendioxidstrom produzierenden Anlage stammen kann. Beispiele für den Kohlendioxidstrom produzierende Anlagen sind Kraftwerke, Zementwerke, Stahlwerke und chemische Produktionsanlagen.

Der Import von Wärme in Form von Dampf weist insbesondere in Bezug auf Methanolanlagen die folgenden Nachteile und Probleme auf:
- Der Dampf muss in ausreichender Menge und zu geeigneten Bedingungen (zum Beispiel bei Drücken von 2 bis 6,5 bar; Temperaturen von mehr als 90 °C, mehr als 120 °C, oder mehr als 150 °C; gesättigt oder überhitzt) in der Nähe der Methanolanlage verfügbar sein.
- Der Kohlendioxid-Fußabdruck des verwendeten Dampfes sollte den Kohlendioxid-Fußabdruck einer kohlendioxidbasierten Methanolanlage nicht verschlechtern, was jedoch häufig der Fall ist - beispielsweise wenn der importierte Dampf durch Abwärme aus einem Verbrennungsprozess und/oder Oxidationsprozess erzeugt wird.
- Die Methanoldestillation kann in Bezug auf die Nutzung von Wärme oder Dampf mit einer Kohlendioxidabscheideanlage, welche in Abhängigkeit der genutzten Technologie ebenfalls Dampf benötigt, konkurrieren. Ein Beispiel für eine solche Technologie ist die chemische Absorption von Kohlendioxid an Aminen. Für die thermische Regeneration der mit Kohlendioxid beladenen Aminlösung wird Dampf benötigt.
- An bestimmten Standorten steht ausschließlich Strom aus erneuerbaren Energiequellen (Solarstrom, Windstrom) zur Verfügung. Dieser erneuerbare Strom muss daher in Wärme umgewandelt werden, beispielsweise mit Hilfe eines elektrischen Dampfkessels. An einem solchen Standort würde die Methanoldestillation mit einer Elektrolyseanlage um den erneuerbaren Strom konkurrieren und somit den Stromverbrauch und die Kapazität der erneuerbaren Stromquelle signifikant erhöhen.

Elektrolyseanlagen setzen grundsätzlich große Mengen an Abwärme frei, allerdings auf einem so niedrigen Temperaturniveau, dass darauf basierend kein Dampf mit einer Temperatur erzeugt werden kann, mit welchem sich eine Methanoldestillation beheizen ließe. Diese Form der Abwärme wird häufig als *low grade heat* bezeichnet. Die Abwärme aus einem Elektrolyseur wird daher häufig entweder in die Atmosphäre abgeleitet, oder mit oder ohne Niedertemperatur-Wärmepumpe bei Temperaturen von insbesondere weniger als 90 °C in ein Fernwärmenetz eingespeist.

Moderne Elektrolyseure benötigen ein aufwändiges Kühlsystem, um die bei dem elektrochemischen Wasseraufspaltungsprozess entstehende Abwärme an die Umgebung abzugeben. Dies erfolgt beispielsweise durch Luftkühler, kühlwasserbetriebene Plattenwärmeübertrager und/oder Kühltürme. Die Nutzung zumindest eines Teils der Abwärme eines Elektrolyseurs würde daher die Betriebskosten (OPEX), die Investitionskosten (CAPEX), sowie den Platzbedarf der Elektrolyseanlage, insbesondere ihres Kühlsystems, reduzieren. Dies kann beispielsweis durch Einsparungen von Zusatzwasser für Kühltürme oder von Strom für Luftkühler erfolgen.

In diesem Zusammenhang offenbart US 11,247,955 B2 ein Verfahren zur Herstellung von Methanol aus einem Kohlendioxidstrom und einem Wasserstoffstrom, in welchem der Wasserstoffstrom durch Elektrolyse von Wasser bereitgestellt wird. Die Abwärme aus dem Elektrolysesystem wird als Wärmequelle für ein Wärmepumpensystem genutzt, und die von dem Wärmepumpensystem abgegebene Wärme wird auf Dampf übertragen, welcher beispielsweise in einem Verdampfer für die Destillation des erzeugten Rohmethanols genutzt wird.

### Beschreibung der Erfindung

Die von der US 11,247,955 B2 beschriebene Vorgehensweise weist den Nachteil auf, dass die von dem Wärmepumpensystem abgegebene Wärme unmittelbar auf Dampf übertragen wird. Dampf als gasförmiges Medium ist für die Aufnahme von Wärme ungünstig, da dessen mengenspezifisches Wärmeaufnahmevermögen weitaus niedriger ist als das einer vergleichbaren Flüssigkeit. So weist beispielsweise Dampf bei 5 bar eine spezifische Wärmekapazität von 2,5 kJ/kg/K auf, während flüssiges Wasser bei 5 bar eine spezifische Wärmekapazität von 4,3 kJ/kg/K aufweist, sodass die zur Übertragung eines definierten Wärmestroms bei einer definierten Temperaturdifferenz benötigte Menge an Dampf höher wäre als die an Wasser. Ferner erfordert ein Dampfstrom vergleichbarer Menge größere Rohrleitungsdurchmesser als eine Flüssigkeit, da Dampf eine um ein Vielfaches geringere Dichte hat als Wasser, beispielsweise 2.7 kg/m³ (Dampf) im Vergleich zu 915 kg/m³ (Wasser). Große Rohrleitungsdurchmesser erhöhen die Kosten der betreffenden Rohrleitung. Sollte die Wärme dem Verbraucher in Form von Dampf bereitgestellt werden, kann mithilfe eines Phasenwechsels der erwärmten Flüssigkeit, nachdem ein Wärmepumpenmedium die Wärme auf diese übertragen hat, mit einer spezifischen Enthalpieänderung von beispielsweise etwa 2100 kJ/kg für Wasser, eine definierte Abwärmemenge unter signifikant geringeren Massenströmen bzw. Temperaturdifferenzen im Wärmeträgermedium genutzt werden als bei der Wärmeübertragung vom Wärmepumpenmedium auf Dampf.

Ebenfalls nachteilig an Dampf (per Definition trocken, gesättigt oder überhitzt) als wärmeaufnehmendes Medium ist der signifikant geringere und damit schlechtere Wärmeübergangskoeffizient α zwischen Wärmeübertragerwand und Medium im Vergleich zu einer zu erwärmenden Flüssigkeit. Entsprechende Wärmeübertrager benötigten entsprechend der Gleichung Q = α * A * ΔT unter Annahme gleicher Temperaturdifferenz ΔT zwischen Wand und flüssigem bzw. gasförmigem Medium eine erheblich höhere Fläche A zum Ausgleich des geringeren α. Die größere Fläche schlägt sich direkt in den Aufwendungen für Bauteile nieder.

Ein auf Basis der PEM-Technologie betriebener Elektrolyseur kann bei Volllast durchaus ein Mehrfaches der für eine Methanolanlage erforderlichen Wärmeimporte als Abwärme freisetzen, entsprechend der für die Methanolsynthese erforderlichen Wasserstoff-Produktionskapazität dieses Elektrolyseurs. Das Problem besteht jedoch wie vorbeschrieben darin, dass die Abwärme regelmäßig bei Temperaturen von nur 40 °C bis 60 °C zur Verfügung steht (*low grade heat*)*.* Der Unterschied zwischen dieser Elektrolyseurabwärmetemperatur und der für die Methanoldestillation benötigten Temperatur von regelmäßig über 120 °C sollte daher bei Verwendung eines Wärmepumpensystems auf möglichst effiziente Art und Weise überwunden werden.

Eine allgemeine Aufgabe der vorliegenden Erfindung besteht somit darin, die Nachteile des Standes der Technik zumindest teilweise zu überwinden.

Insbesondere besteht eine Aufgabe der vorliegenden Erfindung darin, die Abwärme aus einer Elektrolyseanlage mit Hilfe eines Wärmepumpensystems auf möglichst effiziente Weise in einer Destillationsanlage für die Abtrennung von Methanol aus einem methanolhaltigen Strom zu nutzen.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie. Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zur Herstellung von Methanol, umfassend die Verfahrensschritte
a) Bereitstellen eines Synthesegasstroms, welcher zumindest eine Wasserstoffkomponente und eine Kohlenstoffoxidkomponente umfasst, wobei zumindest eine der Komponenten zumindest teilweise durch einen Elektrolyseur bereitgestellt wird;
b) Umsetzen des Synthesegasstroms an einem Methanolsynthesekatalysator in einer Reaktoranordnung zu einem gasförmigen Rohmethanolstrom ;
c) Erzeugen eines flüssigen Rohmethanolstroms durch Kondensieren und Abscheiden des gemäß Schritt b) erhaltenen Rohmethanolstroms;
d) Thermisches Abtrennen von Methanol aus dem flüssigen Rohmethanolstrom in einer Destillationsvorrichtung, wobei die Destillationsvorrichtung zumindest eine Destillationskolonne aufweist, in welcher ein methanolhaltiger Strom in einem Destillationsverdampfer verdampft wird,
dadurch gekennzeichnet, dass
in dem Elektrolyseur erzeugte Abwärme durch indirekte Wärmeübertragung auf ein zirkulierendes Wärmepumpenmedium eines Wärmepumpensystems übertragen wird, wodurch das Wärmepumpenmedium zumindest teilweise verdampft, das Wärmepumpenmedium anschließend verdichtet wird, und das Wärmepumpenmedium durch zumindest teilweises Kondensieren abgegebene Wärme durch indirekte Wärmeübertragung auf ein flüssiges Medium überträgt, und die von dem Wärmepumpensystem auf das flüssige Medium übertragene Wärme zumindest teilweise für die Verdampfung des methanolhaltigen Stroms im Destillationsverdampfer gemäß Schritt d) genutzt wird.

Bei dem methanolhaltigen Strom handelt es sich um einen Strom, welcher zumindest teilweise in flüssiger Phase vorliegt, wobei der flüssige Anteil dieses Stroms im Destillationsverdampfer verdampft wird, vorzugsweise vollständig verdampft wird. Es spielt in Bezug auf den methanolhaltigen Strom keine Rolle, ob dieser teilweise oder vollständig in flüssiger Phase vorliegt. Entscheidend ist alleine, dass zumindest ein Teil der von dem Wärmepumpenmedium abgegebenen Wärme für die Verdampfung des flüssigen Anteils des methanolhaltigen Stroms genutzt wird.

Die Destillationsvorrichtung umfasst eine oder mehrere Destillationskolonnen. Sind mehrere Destillationskolonnen von der Destillationsvorrichtung umfasst, so sind diese vorzugsweise seriell angeordnet. Vorzugsweise erzeugt jede der Destillationskolonnen ein Kopfprodukt und ein Sumpfprodukt, wobei das Kopfprodukt bei geringerem Druck und/oder bei geringerer Temperatur siedet als das Sumpfprodukt.

Der in Schritt c) erzeugte flüssige Rohmethanolstrom wird in der Destillationsvorrichtung mit dem Zweck der Abtrennung von Methanol aus dem Rohmethanolstrom behandelt. Dabei soll insbesondere ein Strom aus reinem Methanol gewonnen werden. In der Destillationsvorrichtung werden ferner Wasser und Nebenprodukte aus dem Rohmethanolstrom abgetrennt.

Der methanolhaltige Strom ist derjenige Strom, welcher im Destillationsverdampfer einer Destillationskolonne verdampft wird. Der einer Destillationskolonne zugeführte Strom (Zulaufstrom) unterscheidet sich in seiner Zusammensetzung von dem methanolhaltigen Strom, da der Destillationsverdampfer insbesondere einen Teil des Sumpfs der jeweiligen Kolonne bildet. Der Zulaufstrom zu einer Destillationskolonne kann als methanolhaltiger Zulaufstrom bezeichnet werden.

Im Falle der ersten der in Serie angeordneten Destillationskolonnen, oder wenn nur eine Destillationskolonne vorhanden ist, entspricht der methanolhaltiger Zulaufstrom in Bezug auf seine Zusammensetzung dem flüssigen Rohmethanolstrom. Der methanolhaltige Zulaufstrom kann hier als erster methanolhaltiger Zulaufstrom bezeichnet werden.

Gemäß einem Beispiel werden in einer ersten Destillationskolonne leicht siedende Nebenprodukte von einem ersten methanolhaltigen Zulaufstrom abgetrennt. Das Sumpfprodukt wird einer zweiten Destillationskolonne als zweiter methanolhaltiger Zulaufstrom zugeführt. Als Kopfprodukt dieser zweiten Destillationskolonne wird im Wesentlichen reines Methanol gebildet, während das Sumpfprodukt im Wesentlichen Wasser enthält, mit einem Methanolgehalt von bis zu einem Volumenprozent. Das Sumpfprodukt der zweiten Destillationskolonne kann zur weiteren Gewinnung von Methanol einer weiteren, dritten Destillationskolonne als dritter methanolhaltiger Zulaufstrom zugeführt werden.

Zumindest eine der Destillationskolonnen der Destillationsvorrichtung kann über einen Seitenabzug für einen methanol- und wasserhaltigen Strom verfügen.

Beispiele für geeignete Wärmepumpenmedien sind Wasser, Methanol, Ethanol, Kohlenwasserstoffe, Oxygenate, halogenierte oder teilhalogenierte Kohlenwasserstoffe, oder Hydrofluorolefine.

Durch die Verdichtung des Wärmepumpenmediums wird dessen Temperatur erhöht. Gemäß einer Ausführungsform kann die Temperatur des Wärmepumpenmediums durch zusätzliches Heizen, beispielsweise durch eine externe Wärmequelle, weiter erhöht werden. Bei der externen Wärmequelle kann es sich um eine elektrische Heizvorrichtung handeln. Nach der Verdichtung gibt das Wärmepumpenmedium Wärme durch indirekte Wärmeübertragung an ein flüssiges Medium ab, wodurch das Wärmepumpenmedium zumindest teilweise kondensiert. Entsprechend dem Funktionsprinzip einer Kompressionswärmepumpe wird der Druck des Wärmepumpenmediums nach der Wärmeübertragung auf das flüssige Medium gesenkt. Dies erfolgt gemäß einer Ausführungsform mittels eines Entspannungsventils. Anschließend beginnt der Zyklus von neuem, das heißt, das Wärmepumpenmedium nimmt Abwärme aus dem Elektrolyseur auf, wodurch es zumindest teilweise verdampft.

Die Wärmerückgewinnung und Wärmeübertragung über das Wärmepumpenmedium in Kombination mit der Verwendung einer Flüssigkeit als Medium, auf welches die Wärme vom Wärmepumpenmedium übertragen wird, ist ein Vorteil gegenüber dem in US 11,247,955 B2 gelehrten Ansatz. Dieser schlägt vor ein Medium zu verwenden welches in Dampfform vorliegt, auf welches die Wärme des Wärmepumpenmediums in einem Wärmeübertrager übertragen wird.

Die von dem Wärmepumpensystem auf das flüssige Medium übertragene Wärme wird zumindest teilweise für die Verdampfung des methanolhaltigen Stroms im Destillationsverdampfer gemäß Schritt d) genutzt. Dass diese Wärme für die Verdampfung des methanolhaltigen Stroms genutzt wird bedeutet nicht zwangsläufig, dass der methanolhaltige Strom durch die Übertragung der Wärme des Wärmepumpenmediums im Destillationsverdampfer vollständig verdampft wird. Durch die Übertragung der Wärme des Wärmepumpenmediums wird die Enthalpie des methanolhaltigen Stroms im Destillationsverdampfer erhöht, jedoch kann der methanolhaltige Strom zumindest teilweise im flüssigen Zustand verbleiben. Die vollständige Verdampfung des methanolhaltigen Stroms kann durch einen nachgeschalteten oder parallel betriebenen, weiteren Destillationsverdampfer oder durch eine weitere Wärmeübertragerstruktur im gleichen Destillationsverdampfer durch ein weiteres Heizmedium, beispielsweise Dampf, erfolgen.

Sowohl die Übertragung der Abwärme des Elektrolyseurs auf das druckentspannte Wärmepumpenmedium, als auch die Übertragung der Wärme des verdichteten Wärmepumpenmediums auf das flüssige Medium kann unmittelbar oder mittelbar erfolgen. In jedem Fall jedoch erfolgt die Wärmeübertragung durch indirekte Wärmeübertragung, das heißt niemals direkt von einem Medium auf das andere, wodurch eine Vermischung der jeweiligen flüssigen oder gasförmigen Medien erfolgen würde.

Unmittelbare Wärmeübertragung (1. Option) im Sinne der Erfindung bedeutet, dass kein weiteres Hilfsmedium für die indirekte Übertragung von Wärme dazwischengeschaltet ist. Die unmittelbare, indirekte Übertragung der Wärme erfolgt beispielsweise in einem Wärmeübertrager von einem Medium auf das andere.

Mittelbare Wärmeübertragung (2. Option) im Sinne der Erfindung bedeutet, dass die indirekte Übertragung der Wärme von dem Wärmepumpenmedium auf ein Hilfsmedium erfolgt, und anschließend vom Hilfsmedium auf das flüssige Medium. Die indirekte Übertragung der Wärme vom Wärmepumpenmedium auf das Hilfsmedium kann mittels eines ersten Wärmeübertragers erfolgen. Die indirekte Übertragung der Wärme vom Hilfsmedium auf das flüssige Medium kann mittels eines zweiten Wärmeübertragers erfolgen. Gemäß einer weiteren Möglichkeit können beide Schritte innerhalb eines Wärmeübertragers erfolgen, welcher über zwei separate Wärmeübertragerstrukturen verfügt.

Ein Vorteil der 1. Option (unmittelbare Wärmeübertragung) ist eine inhärent effizientere Wärmeübertragung durch weniger und kleinere Wärmeübertrager aufgrund der geringeren Zahl an verwendeten Wärmeträgermedien. Ein weiterer Vorteil sind die höheren Temperaturdifferenzen in den Wärmeübertragern, wodurch geringere Durchflussraten des Wärmepumpenmediums innerhalb des Wärmepumpenkreislaufs resultieren.

Ein Vorteil der 2. Option (mittelbare Wärmeübertragung) ist ein erhöhtes Sicherheitsniveau. Zum Beispiel kann im Falle einer Leckage in einem Wärmeübertrager kein Wärmepumpenmedium auf die Prozessseite des Elektrolyseurs oder der Methanol-Destillationsanlage gelangen. Hilfsmedien für die Wärmeübertragung können so gewählt werden, dass sie bei kleinen Leckagen die jeweilige Prozessseite nicht beeinträchtigen. Beispiele für geeignete Hilfsmedien sind Wasser, Wasser-Glykol-Gemische und Thermoöl. Auch im Falle der Wartung der Wärmepumpe ergeben sich durch die 2. Option Vorteile:
- Die Abwärme des Elektrolyseurs kann leichter abgeführt werden, beispielsweise durch Verwendung eines Luftkühlers, der die Kühlung des Hilfsmediums unterstützt;
- Der Wärmebedarf des Destillationsverdampfers kann vorübergehend durch den Import heißer Flüssigkeit oder Dampf in den entsprechenden Wärmeträgerkreislauf gedeckt werden;
- Bei der Verwendung von siedendem Wasser als Hilfsmedium kann das Hilfsmedium mit Dampf gemischt werden, welcher auf der Mantelseite eines wassergekühlten Methanolsynthese-Reaktors anfällt. Gemäß einem Beispiel handelt es sich dabei um Niederdruckdampf.

Der bereitgestellte Synthesegasstrom umfasst zumindest eine Wasserstoffkomponente und eine Kohlenstoffoxidkomponente. Mit anderen Worten, der Synthesegasstrom umfasst Wasserstoff sowie Kohlenmonoxid und/oder Kohlendioxid.

Zumindest eine der Komponenten wird zumindest teilweise durch einen Elektrolyseur bereitgestellt. Insbesondere kann für das Bereitstellen des Synthesegasstroms
- die Wasserstoffkomponente zumindest teilweise elektrolytisch erzeugt werden, oder
- die Kohlenstoffoxid-Komponente zumindest teilweise elektrolytisch erzeugt werden, insbesondere Kohlenmonoxid (CO) elektrolytisch erzeugt werden, oder
- die Wasserstoffkomponente und die Kohlenstoffoxid-Komponente zumindest teilweise elektrolytisch erzeugt werden, insbesondere Wasserstoff und Kohlenmonoxid zumindest teilweise elektrolytisch erzeugt werden.

Gemäß einer bevorzugten Ausführungsform wird die Wasserstoffkomponente vollständig durch einen Elektrolyseur durch Elektrolyse eines wasserhaltigen Elektrolysemediums bereitgestellt. Beispiele für geeignete Verfahren sind die PEM-Elektrolyse, alkalische Elektrolyse, AEM-Elektrolyse, sowie Hochtemperaturelektrolyse mit Dampf als Elektrolysemedium.

Das Verfahren kann auch mehrere Elektrolyseure umfassen, deren Abwärme durch indirekte Wärmeübertragung auf ein zirkulierendes Wärmepumpenmedium eines Wärmepumpensystems übertragen wird.

Gemäß einer weiteren bevorzugten Ausführungsform wird die Wasserstoffkomponente zumindest teilweise durch einen Elektrolyseur durch Elektrolyse eines wasserhaltigen Elektrolysemediums bereitgestellt, und die Kohlenstoffoxid-Komponente wird in Form eines hochkohlendioxidhaltigen Stroms bereitgestellt. Gemäß einem Beispiel enthält der hochkohlendioxidhaltige Strom mindestens 50 Vol.-% Kohlendioxid, vorzugsweise mindestens 75 Vol.-% Kohlendioxid, weiter bevorzugt mindestens 90 Vol.-% Kohlendioxid, weiter bevorzugt mindestens 95 Vol.-% Kohlendioxid, weiter bevorzugt mindestens 99 Vol.-% Kohlendioxid, weiter bevorzugt mindestens 99,5 Vol.-% Kohlendioxid. Diese Art der Methanolsynthese wird auch als CO₂-ba-sierte Methanolsynthese bezeichnet. Der hochkohlendioxidhaltige Strom kann beispielsweise aus einer Kohlenstoffdioxid-Abscheideanlage stammen, beispielsweise aus einer Aminwäsche, einer physikalischen Wäsche (z.B. Methanol-Wäsche), einer Membranabscheideeinrichtung, oder aus einer kryogenen Kohlenstoffdioxid-Abscheideanlage.

Gemäß einer weiteren Ausführungsform wird die Kohlenstoffoxidkomponente des Synthesegasstrom zumindest teilweise durch Elektrolyse eines kohlendioxidhaltigen Stroms bereitgestellt. Durch Elektrolyse von Kohlendioxid wird dieses zumindest teilweise zu Kohlenmonoxid reduziert. Das resultierende, kohlenmonoxidhaltige Gas kann als Kohlenstoffoxid-Komponente für die Methanolsynthese verwendet werden.

Der Synthesegasstrom wird an einem geeigneten Methanolsynthesekatalysator, beispielsweise auf Kupfer-Basis, in einer Reaktoranordung zu einem gasförmigen Rohmethanolstrom umgesetzt. Die Reaktoranordnung kann einen oder mehrere Reaktoren, die in Serie und/oder parallel geschaltet sind, umfassen. Gemäß einer Ausführungsform umfasst die Reaktoranordnung mehrere in Serie geschaltete Reaktoren, wobei nach jedem der Reaktoren eine Zwischenkondensation des Rohmethanolstroms erfolgt, das heißt Schritt c) wird jeweils stromabwärts eines Reaktors dieser Reaktorkaskade durchgeführt. Bei den Reaktoren kann es sich grundsätzlich um wassergekühlte und/oder gasgekühlte Reaktoren handeln, unabhängig von deren Anordnung.

Der Rohmethanolstrom enthält zumindest Methanol und Wasser. Weiterhin sind regelmäßig unerwünschte Nebenprodukte im Rohmethanolstrom vorhanden, welche im thermischen Trennschritt d) mit abgetrennt werden können.

Durch Kondensieren und Abscheiden des gemäß Schritt b) erhältlichen gasförmigen Rohmethanolstroms wird gemäß Schritt c) ein flüssiger Rohmethanolstrom erhalten. Bei nicht kondensierten Bestandteilen kann es sich beispielsweise um nicht umgesetzte Bestandteile des Synthesegasstroms handeln, welche im Falle einer konventionellen Methanolsynthese mit Synthese-Loop zum Einlass der Reaktoranordnung zurückgeführt werden.

Der thermische Trennschritt d) dient insbesondere der Abtrennung von Methanol aus dem flüssigen Rohmethanolstrom. Dabei fallen insbesondere zumindest ein Reinmethanolstrom, zumindest ein Wasserstrom, sowie zumindest ein Nebenproduktstrom an. Dafür wird ein methanolhaltiger Strom in einem Destillationsverdampfer zumindest einer Destillationskolonne verdampft. Die Destillationskolonne oder mehrere Destillationskolonnen bilden einen Teil der Destillationsvorrichtung. Das thermische Trennen gemäß Schritt d) kann somit mehrere hintereinander geschaltete Trennschritte umfassen.

Die bei der Verdampfung in den methanolhaltigen Strom eingetragene Wärme wird durch den Destillationsprozess im Wesentlichen auf das Kopfprodukt der entsprechenden Destillationskolonne übertragen, welchem durch ein Kühlmittel Wärme entzogen wird. Beispiele für Zusammensetzungen des methanolhaltigen Stroms sind
- ein stabilisiertes, das heißt im Wesentlichen von leichtsiedenden Komponenten befreites Rohmethanol im Sumpf einer Vorlaufkolonne als erster Destillationskolonne, und
- ein Gemisch mit Methanol und Wasser als Hauptkomponenten im Sumpf einer Atmosphärischen Kolonne als zweiter Destillationskolonne mit einem geringeren Methanolgehalt als jener im Rohmethanolstrom.

Eine bevorzugte Ausführungsform des erfindungsgemäßen Verfahrens ist dadurch gekennzeichnet, dass von dem Wärmepumpenmedium abgegebene Wärme im Destillationsverdampfer unmittelbar durch indirekte Wärmeübertragung auf den methanolhaltigen Strom übertragen wird, wobei der methanolhaltige Strom das flüssige Medium umfasst.

Diese bevorzugte Ausführungsform ist ein Beispiel für eine unmittelbare Übertragung der von dem Wärmepumpenmedium abgegebenen Wärme auf ein flüssiges Medium. Das flüssige Medium ist gemäß dieser Ausführungsform der methanolhaltige Strom oder ist der flüssige Anteil des methanolhaltigen Stroms, welcher im Destillationsverdampfer verdampft wird. Gemäß einer Ausführungsform wird der methanolhaltige Strom durch die Übertragung der Wärme des Wärmepumpenmediums im Destillationsverdampfer zumindest teilweise verdampft.

Gemäß einer Ausführungsform wird der methanolhaltige Strom durch die Übertragung der Wärme des Wärmepumpenmediums im Destillationsverdampfer nicht vollständig verdampft. Gemäß letzterer Ausführungsform wird der methanolhaltige Strom nach teilweiser Verdampfung mittels der Wärmezufuhr durch das Wärmepumpenmedium anschließend (in Serie) durch ein weiteres Wärmeübertragungsmedium, wie beispielsweise Dampf, zur Vollständigkeit verdampft.

Gemäß einer weiteren Ausführungsform wird nicht der gesamte methanolhaltige Strom durch die Übertragung der Wärme des Wärmepumpenmediums im Destillationsverdampfer verdampft. Gemäß letzterer Ausführungsform wird der nicht mithilfe des Wärmepumpenmediums erhitzte Anteil des methanolhaltigen Stroms parallel durch ein weiteres Wärmeübertragungsmedium, wie beispielsweise Dampf, in einem weiteren Wärmeübertrager verdampft.

Im diesem Zusammenhang spielt es keine Rolle, dass die von dem Wärmepumpenmedium übertragene Wärme teilweise auch auf gasförmiges Rohmethanol übertragen werden kann. Entscheidend ist, dass die von dem Wärmepumpenmedium bereitgestellte Wärme zumindest teilweise auf den methanolhaltigen Strom übertragen wird, welcher dadurch zumindest teilweise verdampft.

Bei dem methanolhaltigen Strom kann es sich um einen Flüssigkeitsstrom handeln, welcher bereits einem Destillationsschritt unterzogen wurde. Entscheidend ist alleine, dass der methanolhaltige Strom Methanol sowie Wasser und/oder Nebenprodukte enthält, so dass eine weitere Destillation und damit Verdampfung in einem Destillationsverdampfer erforderlich sein kann, um einen Methanolstrom mit höherer Reinheit in Bezug auf den Methanol-Gehalt dieses Stroms zu erhalten, oder auch um einen Wasserstrom mit höherer Reinheit in Bezug auf den Wasser-Gehalt dieses Stroms zu erhalten. Bei dem methanolhaltigen Strom kann es sich um einen Strom mit hohem Methanolgehalt und niedrigem Wassergehalt handeln. In diesem Fall kann eine weitere Destillation erforderlich sein kann, um einen Methanolstrom mit höherer Reinheit in Bezug auf den Methanolgehalt dieses Stroms zu erhalten. Bei dem methanolhaltigen Strom kann es sich ferner um einen Strom mit hohem Wassergehalt und niedrigem Methanolgehalt handeln. In diesem Fall kann eine weitere Destillation erforderlich sein, um einen Wasserstrom mit höherer Reinheit in Bezug auf den Wasser-Gehalt dieses Stroms zu erhalten.

Eine bevorzugte Ausführungsform des erfindungsgemäßen Verfahrens ist dadurch gekennzeichnet, dass von dem Wärmepumpenmedium abgegebene Wärme durch indirekte Wärmeübertragung auf ein erstes Hilfsmedium übertragen wird, wobei das erste Hilfsmedium das flüssige Medium umfasst, und von dem ersten Hilfsmedium aufgenommene Wärme durch indirekte Wärmeübertragung im Destillationsverdampfer auf den methanolhaltigen Strom übertragen wird.

Durch die Übertragung von Wärme des Wärmepumpenmediums auf das erste Hilfsmedium kann letzteres entweder flüssig bleiben, oder infolge der Wärmeübertragung zumindest teilweise verdampfen. Für die Übertragung von Wärme vom ersten Hilfsmedium im Destillationsverdampfer auf den methanolhaltigen Strom gilt das oben gesagte. Der methanolhaltige Strom muss nicht zwangsläufig vollständig verdampft werden. Entscheidend für diese Ausführungsform ist alleine, dass zumindest ein Teil der Wärme des Wärmepumpenmediums auf das erste Hilfsmedium übertragen wird, und zumindest ein Teil dieser vom ersten Hilfsmedium aufgenommenen Wärme auf den methanolhaltigen Strom übertragen wird, so dass diese Wärme für die Verdampfung des methanolhaltigen Stroms genutzt werden kann. Die durch das erste Hilfsmedium aufgenommene Wärme kann auch in mehreren Destillationsverdampfern auf mehrere methanolhaltige Ströme übertragen werden.

Bei dem ersten Hilfsmedium handelt es sich gemäß einer Ausführungsform um Wasser mit einem Druck von mindestens 3 bar, vorzugsweise von mehr als 5 bar, jedoch nicht mehr als 90 bar. Das Wasser kann entweder erwärmt werden und dann zum Destillationsverdampfer geführt werden, oder es kann in einem Druckbehälter verdampft werden, um Sattdampf zu erzeugen. Ein derart erzeugter Dampf wird anschließend zum Destillationsverdampfer geführt. Optional kann der so erzeugte Sattdampfstrom verdichtet und wieder gesättigt werden, um dessen Temperatur und Druck zu erhöhen.

Gemäß einer weiteren Ausführungsform handelt es sich bei dem ersten Hilfsmedium um Thermoöl.

Eine bevorzugte Ausführungsform des erfindungsgemäßen Verfahrens ist dadurch gekennzeichnet, dass die Reaktoranordnung einen wassergekühlten Reaktor beinhaltet, und in einem Kühlsystem des wassergekühlten Reaktors ein Dampfstrom erzeugt wird, und der Dampfstrom zumindest teilweise mit dem ersten Hilfsmedium zusammengeführt wird, bevor von dem ersten Hilfsmedium aufgenommene Wärme durch indirekte Wärmübertragung im Destillationsverdampfer auf den methanolhaltigen Strom übertragen wird.

Gemäß dieser bevorzugten Ausführungsform wird die Wärmeintegration des Gesamtsystems verbessert, wenn die Reaktoranordnung zumindest einen wassergekühlten Reaktor aufweist. Üblicherweise wird für die indirekte Kühlung des Katalysatorbetts im Methanolsynthesereaktor siedendes Kesselspeisewasser als Kühlmedium verwendet, um sich das Prinzip der Siedekühlung zunutze zu machen. Der dabei auf der Mantelseite des Reaktors erzeugte Dampfstrom, insbesondere Niederdruckdampfstrom, kann zumindest teilweise mit dem ersten Hilfsmedium zusammengeführt werden, bevor die von dem ersten Hilfsmedium aufgenommene Wärme durch indirekte Wärmübertragung im Destillationsverdampfer auf den methanolhaltigen Strom übertragen wird. Mit anderen Worten, das erste Hilfsmedium wird nach der Übertragung von Wärme vom Wärmepumpendmedium auf das erste Hilfsmedium durch den vorgenannten Dampfstrom ergänzt, und anschließend erfolgt die Übertragung der Wärme vom ersten Hilfsmedium auf den methanolhaltigen Strom im Destillationsverdampfer. Dabei wird nicht nur von dem ersten Hilfsmedium aus dem Wärmepumpenmedium aufgenommene Wärme übertragen, sondern auch im Dampfstrom enthaltene Wärme übertragen.

Eine bevorzugte Ausführungsform des erfindungsgemäßen Verfahrens ist dadurch gekennzeichnet, dass in dem Elektrolyseur erzeugte Abwärme unmittelbar durch indirekte Wärmeübertragung von einem Elektrolysemedium auf das Wärmepumpenmedium übertragen wird.

Diese bevorzugte Ausführungsform entspricht einem Beispiel, in welchem im Elektrolyseur erzeugte Abwärme unmittelbar durch indirekte Wärmeübertragung auf das Wärmepumpenmedium übertragen wird, was mit den oben erläuterten Vorteilen verbunden ist.

Die in dem Elektrolyseur erzeugte Abwärme ist insbesondere im Elektrolysemedium gespeichert, welches nach erfolgter Elektrolysereaktion aus dem Elektrolysezellenstapel des Elektrolyseurs austritt. Abwärme dieses Mediums wird unmittelbar oder mittelbar durch indirekte Wärmeübertragung auf das Wärmepumpenmedium übertragen, wodurch letzteres zumindest teilweise verdampft. Die Übertragung von Wärme des Elektrolysemediums, also zumindest eines Teils der Abwärme des Elektrolyseurs, kann vor oder nach der Abtrennung von Produktgasen aus dem Elektrolysemedium erfolgen, soll jedoch vorzugsweise erfolgen, bevor das an Produktgasen verarmte Elektrolysemedium wieder in den Elektrolysezellenstapel eintritt.

Eine bevorzugte Ausführungsform des erfindungsgemäßen Verfahrens ist dadurch gekennzeichnet, dass in dem Elektrolyseur erzeugte Abwärme durch indirekte Wärmeübertragung auf ein zweites Hilfsmedium übertragen wird, und von dem zweiten Hilfsmedium aufgenommene Wärme auf das Wärmepumpenmedium übertragen wird.

Diese bevorzugte Ausführungsform entspricht einem Beispiel, in welchem im Elektrolyseur erzeugte Abwärme mittelbar durch indirekte Wärmeübertragung auf das Wärmepumpenmedium übertragen wird. Gemäß dieser bevorzugten Ausführungsform erfolgt die Übertragung von in einem Elektrolysemedium enthaltener Abwärme zunächst indirekt auf ein zweites Hilfsmedium, und anschließend durch indirekte Wärmeübertragung vom zweiten Hilfsmedium auf das Wärmepumpenmedium.

Eine bevorzugte Ausführungsform des erfindungsgemäßen Verfahrens ist dadurch gekennzeichnet, dass die Reaktoranordnung einen wassergekühlten Reaktor beinhaltet, und in einem Kühlsystem des wassergekühlten Reaktors ein Dampfstrom erzeugt wird, und der Dampfstrom zumindest teilweise mit dem zweiten Hilfsmedium zusammengeführt wird, bevor von dem zweiten Hilfsmedium aufgenommene Wärme auf das Wärmepumpenmedium übertragen wird.

Gemäß dieser bevorzugten Ausführungsform wird die Wärmeintegration des Gesamtsystems analog der oben beschriebenen Ausführungsform verbessert, wenn die Reaktoranordnung zumindest einen wassergekühlten Reaktor aufweist. Hier erfolgt dies jedoch in Bezug auf die Übertragung von Wärme auf das Wärmepumpenmedium, so dass dieses zumindest teilweise verdampft. Gemäß dieser Ausführungsform wird der auf der Mantelseite eines wassergekühlten Reaktors erzeugte Dampfstrom, insbesondere Niederdruckdampfstrom, zumindest teilweise mit dem zweiten Hilfsmedium zusammengeführt, bevor die von dem zweiten Hilfsmedium von dem Elektrolyseur aufgenommene Abwärme durch indirekte Wärmübertragung auf das Wärmepumpenmedium übertragen wird, so dass dieses zumindest teilweise verdampft. Mit anderen Worten, das zweite Hilfsmedium wird durch den vorgenannten Dampfstrom ergänzt, und anschließend erfolgt die Übertragung der Wärme vom zweiten Hilfsmedium auf das Wärmepumpenmedium. Dabei wird nicht nur von dem Elektrolyseur erzeugte Abwärme auf das Wärmepumpenmedium übertragen, sondern auch im Dampfstrom enthaltene Wärme übertragen.

Eine bevorzugte Ausführungsform des erfindungsgemäßen Verfahrens ist dadurch gekennzeichnet, dass das Wärmepumpenmedium Wasser beinhaltet, wobei für das Verdichten des Wärmepumpenmediums ein Dampfstrom genutzt wird.

Insbesondere ist bevorzugt, dass die Reaktoranordnung einen wassergekühlten Reaktor beinhaltet, und in einem Kühlsystem des wassergekühlten Reaktors ein Dampfstrom erzeugt wird, welcher zumindest teilweise für das Verdichten des Wärmepumpenmediums genutzt wird.

Dabei ist weiter bevorzugt, dass das Wärmepumpensystem zum Verdichten des Wärmepumpenmediums eine Treibmittelpumpe beinhaltet, wobei die Treibmittelpumpe einen Saugstutzen, einen Druckstutzen, und einen Treibmedienanschluss aufweist, und wobei der Treibmittelpumpe über den Saugstutzen das zu verdichtende Wärmepumpenmedium zugeführt wird, über den Treibmedienanschluss der Dampfstrom zugeführt wird, und das verdichtete Wärmepumpenmedium über den Druckstutzen aus der Treibmittelpumpe ausgeleitet wird.

Wird Wasser oder zumindest ein wasserhaltiges Medium als Wärmepumpenmedium genutzt, kann ein Dampfstrom zum Verdichten zumindest eines Teils des Wärmepumpenmediums verwendet werden. In diesem Fall ist keine oder weniger elektrische Energie zum Verdichten dieses Teils des Wärmepumpenmediums, beispielsweise mittels eines Kolbenverdichters oder Turboverdichters, erforderlich.

Vorzugsweise wird dafür zumindest ein Teil des Dampfstroms genutzt, welcher im Kühlsystem eines wassergekühlten Reaktors der Reaktoranordnung erzeugt wird.

Weiter bevorzugt erfolgt das Verdichten zumindest eines Teils des Wärmepumpenmediums mittels des Dampfstroms in einer Treibmittelpumpe. Die Treibmittelpumpe kann alternativ auch als Strahlpumpe bezeichnet werden. Da die Treibmittelpumpe gemäß dieser Ausführungsform eine verdichtende Wirkung hat, kann diese auch als Injektor bezeichnet werden. Die Treibmittelpumpe verfügt über einen Treibmedienanschluss, einen Saugstutzen, und über einen Druckstutzen. Über den Treibmedienanschluss wird der Dampfstrom, über den Saugstutzen das druckentspannte Wärmepumpenmedium in die Treibmittelpumpe eingeleitet. Mit Hilfe des Dampfstroms wird der Druck des Wärmepumpenmediums in der Treibmittelpumpe erhöht, das Wärmepumpenmedium somit durch den Dampfstrom verdichtet. Verdichtetes Wärmepumpenmedium wird über den Druckstutzen aus der Treibmittelpumpe ausgeleitet. Der Druck am Treibmedienanschluss ist dabei höher als der Druck am Druckstutzen, und der Druck am Druckstutzen wiederum höher als der Druck am Saugstutzen. Da die Wärmepumpenmedienmenge durch die Zuführung des Dampfs am Treibmedienanschluss derTreibmittelpumpe erhöht wird, wird ein Teilstrom des Wärmepumpenmediums vorzugsweise stromaufwärts der Position der Wärmeübertragung auf das flüssige Medium aus dem Wärmepumpenmedienkreislauf abgezweigt. Da es sich dabei insbesondere um kondensiertes Wasser handelt, wird dieses vorzugsweise dem Kühlsystem des wassergekühlten Reaktors der Reaktoranordnung als Kühlwasser zugeführt. Dadurch wird der Kreislauf zwischen wassergekühltem Reaktor und zirkulierendem Wärmepumpenmedium geschlossen.

Wird Wasser oder zumindest ein wasserhaltiges Medium als Wärmepumpenmedium genutzt, kann die Verdichtung des Wärmepumpenmediums durch Kombination von einer Treibmittelpumpe und eines Verdichters erfolgen. Dabei können die Treibmittelpumpe und der Verdichter nacheinander oder parallel geschaltet sein.

Vorzugsweise wird dafür zumindest ein Teil des Dampfstroms genutzt, welcher im Kühlsystem eines wassergekühlten Reaktors der Reaktoranordnung erzeugt wird.

Eine bevorzugte Ausführungsform des erfindungsgemäßen Verfahrens ist dadurch gekennzeichnet, dass das Wärmepumpensystem als Hochtemperaturwärmepumpensystem ausgebildet ist, in welchem das verdichtete Wärmepumpenmedium eine Temperatur von mindestens 60 °C aufweist, bevorzugt eine Temperatur von mindestens 100 °C aufweist.

Weiter bevorzugt weist das Wärmepumpenmedium eine Temperatur von höchstens 200 °C auf, bevorzugt von höchstens 180 °C.

Eine bevorzugte Ausführungsform des erfindungsgemäßen Verfahrens ist dadurch gekennzeichnet, dass die Kohlenstoffoxidkomponente des Synthesegasstroms zumindest 50 mol-% Kohlendioxid umfasst, und zumindest ein Teil der Kohlenstoffoxid-komponente durch ein Kohlenstoffdioxid-Abscheideverfahren erzeugt wird.

Weiter bevorzugt umfasst die Kohlenstoffoxidkomponente des Synthesegasstroms mindestens 75 Vol.-% Kohlendioxid, weiter bevorzugt mindestens 90 Vol.-% Kohlendioxid, weiter bevorzugt mindestens 95 Vol.-% Kohlendioxid, weiter bevorzugt mindestens 99 Vol.-% Kohlendioxid, weiter bevorzugt mindestens 99,5 Vol.-% Kohlendioxid.

Unter Kohlendioxid-Abscheideverfahren ist dabei ein Verfahren zu verstehen, welches mehr oder weniger selektiv Kohlendioxid aus einem Gasgemischstrom abtrennt. Die Abtrennung kann durch ein kryogenes Verfahren erfolgen, welches eine Kondensation und gegebenenfalls eine kryogene Destillation des Kohlendioxids umfasst. Weiterhin kann ein solches Verfahren einen oder mehrere durch Membranen bewirkte Trennschritte umfassen. Weiterhin kann die Abtrennung ein absorptives Verfahren umfassen, welches wenigstens einen physikalischen oder chemischen Absorptionsschritt, sowie einen davon gefolgten Desorptionsschritt umfasst. Weiterhin kann die Abscheidung ein adsorptives Verfahren umfassen, welches wenigstens einen Adsorptionsschritt an einer festen Oberfläche und einen davon gefolgten Desorptionsschritt von dieser Oberfläche umfasst. Dabei muss nicht zwangsläufig das Kohlendioxid selbst diejenige Spezies sein, welche an der jeweiligen Oberfläche adsorptiv bindet. Weiterhin kann die Abscheidung ein Membran-basiertes Verfahren umfassen, welches wenigstens einen Retentatstrom und einen Permeatstrom produziert, von denen zumindest ein Strom einen höheren Kohlendioxidgehalt aufweist als der der Membran zugeführte Einsatzstrom.

### Ausführungsbeispiele

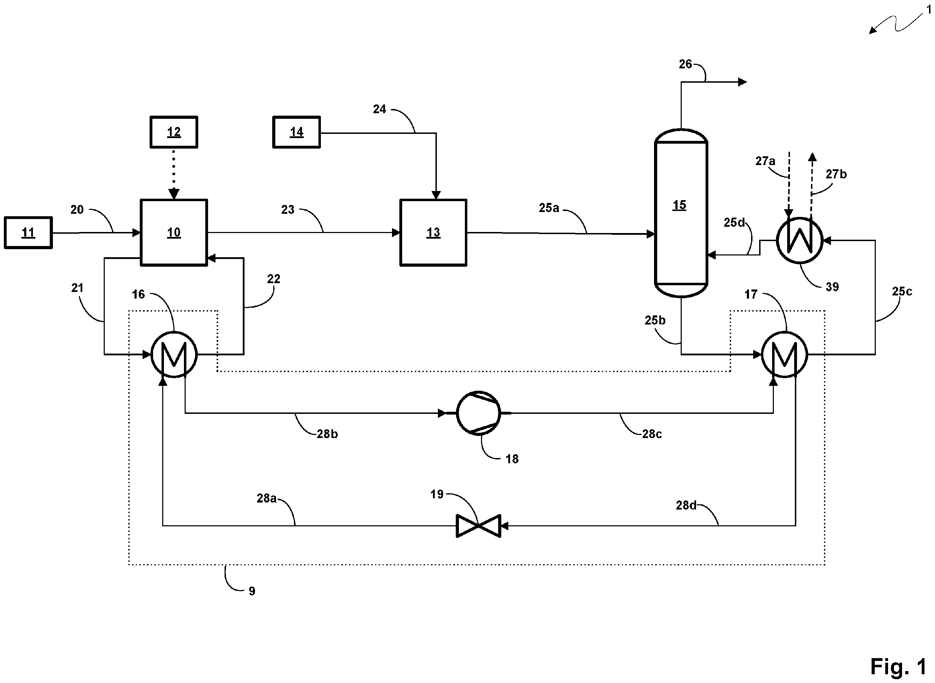

Die Erfindung wird im Folgenden durch drei Ausführungsbeispiele und ein Zahlenbeispiele näher erläutert. Die Ausführungsbeispiele sind durch die Figuren 1 bis 3 illustriert. In den Figuren sind funktionell und/oder strukturell gleiche oder zumindest ähnliche Bestandteile mit gleichen Bezugszeichen versehen.

Es zeigt
- Figur 1: ein vereinfachtes Blockfließbild eines ersten Beispiels des Verfahrens gemäß der Erfindung,
- Figur 2: ein vereinfachtes Blockfließbild eines zweiten Beispiels des Verfahrens gemäß der Erfindung,
- Figur 3: ein vereinfachtes Blockfließbild eines dritten Beispiels des Verfahrens gemäß der Erfindung.

Figur 1 zeigt ein Blockfließbild eines Verfahrens 1 gemäß einem ersten Beispiel der Erfindung.

Im Beispiel gemäß Figur 1 erfolgt sowohl die Übertragung der Abwärme eines Elektrolyseurs auf das Wärmepumpenmedium, als auch die Übertragung der Kondensationswärme des Wärmepumpenmediums auf den methanolhaltigen Strom unmittelbar durch indirekte Wärmeübertragung, das heißt ohne Wärmeübertrager-Hilfsmedium.

Ein Elektrolyseur 10, beispielsweise ein PEM-Elektrolyseur, wird über ein Wasserreservoir 11 mit einem Wasserstrom 20 und über eine Gleichstromquelle 12 mit elektrischer Energie versorgt. Durch elektrochemische Spaltung von Wasser als Elektrolysemedium erzeugt der Elektrolyseur einen Sauerstoffstrom (nicht gezeigt) und einen Wasserstoffstrom 23. Der Sauerstoffstrom wird an die Atmosphäre abgegeben. Alternativ könnte der Sauerstoffstrom auch an einen anderen Prozess oder an eine andere Stelle der Methanolanlage abgegeben werden. Der Wasserstoffstrom 23 wird einer Methanolsynthese-Reaktoranordnung 13 zugeführt, welche zumindest einen wassergekühlten Methanolsynthesereaktor, einen Kühler, einen Kondensator, und einen Abscheider enthält (Details nicht gezeigt). Der Methanolsynthese-Reaktoranordnung 13 wird ferner ein Kohlendioxidstrom 24 aus einer Kohlendioxidquelle 14 zugeführt. Bei der Kohlendioxidquelle 14 handelt es sich um die Regenerationskolonne einer Aminwäsche-Einheit. In der Methanolsynthese-Reaktoranordnung 13 wird Wasserstoff des Wasserstoffstroms 23 und Kohlendioxid des Kohlendioxidstroms 24 an einem Methanolsynthesekatalysator zu einem Rohmethanolstrom umgesetzt. Der Rohmethanolstrom, welcher zumindest Methanol und Wasser enthält, wird zunächst einer Vorlaufkolonne zugeführt, in welcher leichtsiedende Komponenten als Kopfprodukt entfernt werden (nicht gezeigt). Das Sumpfprodukt der Vorlaufkolonne wird als methanolhaltiger Zulaufstrom 25a einer Destillationskolonne 15 zugeführt, in welcher ein Methanolstrom 26 als Kopfprodukt, sowie ein Wasserstrom (nicht gezeigt) als Sumpfprodukt erzeugt wird.

Das Verfahren beinhaltet ferner ein Wärmepumpensystem 9, welches einen Wärmeübertrager 16, einen Wärmeübertrager 17, einen Kompressor 18, sowie ein Drosselventil 19. Die vorgenannten Komponenten sind durch entsprechende Leitungen fluidisch miteinander verbunden, so dass zwischen den genannten Komponenten ein Wärmepumpenmedium zirkulieren kann. Der Wärmeübertrager 16 weist die Funktion des Verdampfers des Wärmepumpensystems auf, der Wärmeübertrager 17 die Funktion des Verflüssigers.

Aus dem Elektrolyseur 10 wird ein Strom eines heißen Elektrolysemediums 21 ausgeleitet, dessen Wärme im Wärmeübertrager 16 teilweise auf ein in diesen Wärmeübertrager 16 eintretendes, flüssiges Wärmepumpenmedium 28a übertragen wird. Das Wärmepumpenmedium 28a wird dadurch verdampft. Das heiße Elektrolysemedium 21 wird entsprechend gekühlt, wodurch ein Strom eines gekühlten Elektrolysemediums 22 aus dem Wärmeübertrager 16 ausgeleitet und zum Elektrolyseur zurückgeführt wird. Das verdampfte Wärmepumpenmedium 28b wird dem Kompressor 18 zugeführt, wodurch die Temperatur und der Druck des verdampften Wärmepumpenmediums erhöht wird. Das verdichtete Wärmepumpenmedium 28c wird dem Wärmeübertrager 17 zugeführt, in welchem das Wärmepumpenmedium in Wärmeaustausch mit dem aus der Destillationskolonne 15 abgezogenen methanolhaltigen Strom 25b verflüssigt (kondensiert) wird. Der Wärmeübertrager 17 bildet in diesem Fall nicht nur einen Teil des Wärmepumpensystems 9, sondern auch einen Teil des Destillationsverdampfers der Destillationskolonne 15. Im Wärmeübertrager 17 wird der methanolhaltige Strom 25b durch die Übertragung der Kondensationswärme des Wärmepumpenmediums teilweise, jedoch nicht vollständig verdampft. Für die vollständige Verdampfung wird der teilverdampfte methanolhaltige Strom 25c einem weiteren Wärmeübertrager 39 zugeführt, welcher ebenfalls einen Teil des Destillationsverdampfers der Destillationskolonne 15 bildet. Der Wärmeübertrager 39 wird mit einem Dampfstrom 27a versorgt, durch welchen der methanolhaltige Strom 25c vollständig verdampft wird. Der vollständig verdampfte methanolhaltige Strom 25d tritt in die Destillationskolonne 15 ein, um dort thermisch in einen Methanolstrom 26 und einen Wasserstrom (nicht gezeigt) getrennt zu werden. Im Wärmeübertrager 39 kondensiert der Dampfstrom 27a, und verlässt den Wärmeübertrager 39 als Kondensatstrom 27b.

Der Strom des kondensierten, abgekühlten Wärmepumpenmediums 28d wird über das Drosselventil 19 entspannt und als druckentspannter Wärmepumpenmedienstrom 28a dem Wärmeübertrager 16 (Verdampfer) zugeführt, in welchem das Wärmepumpenmedium erneut durch Aufnahme der Abwärme des Elektrolyseurs 10 verdampft wird.

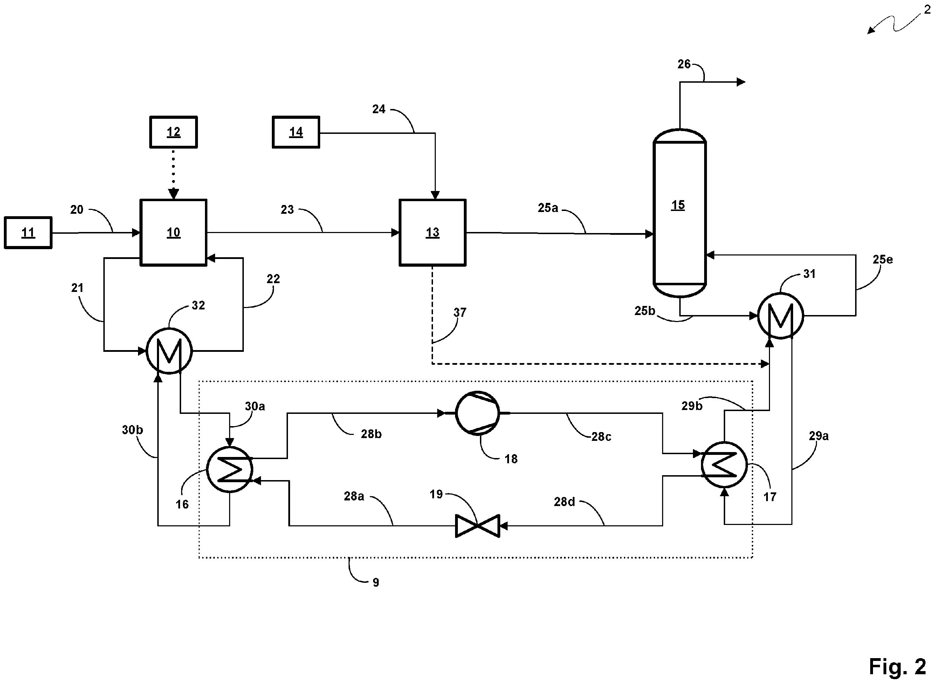

Figur 2 zeigt ein Blockfließbild eines Verfahrens 2 gemäß einem zweiten Beispiel der Erfindung.

Im Beispiel gemäß Figur 2 erfolgt sowohl die Übertragung der Abwärme des Elektrolyseurs auf das Wärmepumpenmedium, als auch die Übertragung der Kondensationswärme des Wärmepumpenmediums auf den methanolhaltigen Strom mittelbar durch indirekte Wärmeübertragung, das heißt jeweils unter Nutzung eines Wärmeübertrager-Hilfsmediums mit zwischengeschalteten Wärmeübertragern.

Im Folgenden wird im Wesentlichen auf Unterschiede zum Beispiel gemäß Figur 1 eingegangen.

Die Übertragung der Abwärme des Elektrolyseurs 10 erfolgt zunächst in einem Wärmeübertrager 32, welchem ein Strom eines abgekühlten zweiten Hilfsmediums 30b zugeführt wird. Wärme des Stroms des heißen Elektrolysemediums 21 wird im Wärmeübertrager 32 auf den Strom des abgekühlten Hilfsmediums 30b übertragen. Der dadurch erwärmte Strom des Hilfsmediums 30a wird aus dem Wärmeübertrager 32 ausgeleitet und bewirkt im Wärmeübertrager 16 (Verdampfer) die Verdampfung des diesem Wärmeübertrager zugeführten Wärmepumpenmediums 28a, welches anschließend als verdampftes Wärmepumpenmedium 28b dem Kompressor 18 zugeführt wird.

Auf der Verflüssiger-Seite des Wärmepumpensystems 9 bewirkt ein abgekühlter Strom eines ersten Hilfsmediums 29a die Kondensation des verdichteten Wärmepumpenmedienstroms 28c. Der daraus resultierende, erwärmte Hilfsmedienstrom 29b wird mit einem Dampfstrom 37 zusammengeführt, welcher dem Kühlsystem eines wassergekühlten Reaktors der Methanolsynthese-Reaktoranordnung 13 entnommen wird. Ein entsprechend kombinierter Strom aus Dampf und erwärmtem erstem Hilfsmedium wird einem Wärmeübertrager 31 zugeführt, in welchem die Verdampfung des methanolhaltigen Stroms 25b bewirkt wird. Der verdampfte methanolhaltige Strom 25e wird der Destillationskolonne 15 zur thermischen Trennung in den Methanolstrom 26 und Wasserstrom (nicht gezeigt) zugeführt. Der Wärmeübertrager 31 ist hier der Destillationsverdampfer der Destillationskolonne 15 und bildet keinen Teil des Wärmepumpensystems 9.

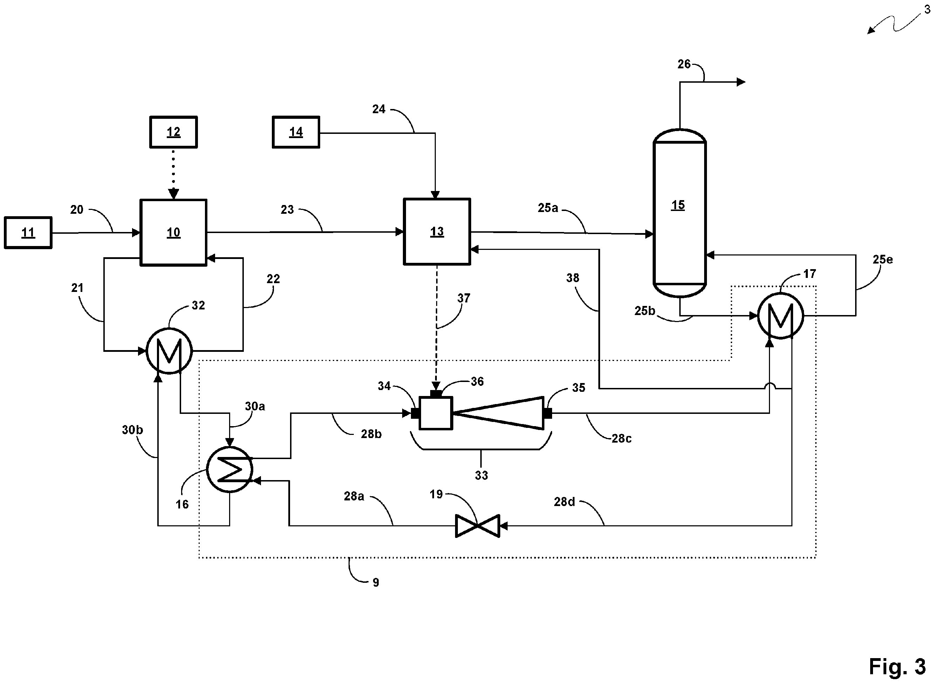

Figur 3 zeigt ein Blockfließbild eines Verfahrens 3 gemäß einem dritten Beispiel der Erfindung.

Im Beispiel gemäß Figur 3 erfolgt die Übertragung der Abwärme des Elektrolyseurs analog dem Beispiel der Figur 2 mit Hilfe eines zweiten Hilfsmediums zunächst im Wärmeübertrager 32 auf das zweite Hilfsmedium, und anschließend im Wärmeübertrager 16 vom zweiten Hilfsmedium auf das druckentspannte Wärmepumpenmedium 28a, welches dadurch verdampft.

Im Beispiel gemäß Figur 3 wird als Wärmepumpenmedium Wasser verwendet.

Der Strom des verdampften Wärmepumpenmediums 28b wird über einen Saugstutzen 34 einer Treibmittelpumpe 33 zugeführt. Der Treibmittelpumpe wird weiterhin über einen Treibmedienanschluss 36 ein Dampfstrom 37 zugeführt. Der Dampfstrom 37 wird dem Kühlsystem eines wassergekühlten Reaktors der Methanolsynthese-Reaktoranordnung 13 entnommen. Durch den Dampfstrom 37 wird das dampfförmige Wärmepumpenmedium 28b in der Treibmittelpumpe 33 verdichtet und als Strom eines verdichteten Wärmepumpenmediums 28c über einen Druckstutzen 35 der Treibmittelpumpe 33 aus dieser ausgeleitet. Das verdichtete Wärmepumpenmedium 28c kondensiert im Wärmeübertrager 17 und erhitzt dadurch einen methanolhaltigen Strom 25b, welcher dadurch verdampft und als gasförmiger methanolhaltiger Strom 25e in die Destillationskolonne 15 eintritt.

Von dem Strom des kondensierten Wärmepumpenmediums 28d wird ein Teilstrom 38 zwecks Massenbilanzausgleich abgezweigt. Dieser Teilstrom 38 wird dem Kühlsystem des wassergekühlten Reaktors der Methanolsynthese-Reaktoranordnung 13 zur erneuten Verwendung als Kühlwasser zugeführt.

Das folgende Zahlenbeispiel, welches auf Simulationsdaten basiert, soll ferner die Vorteile des erfindungsgemäßen Verfahrens illustrieren.

Ein typischer 100 MW PEM-Elektrolyseur erzeugt bei Volllast eine Wärmemenge von circa 20 bis 25 MW bei ca. 50 °C. Eine Methanolanlage, welche auf Basis von Kohlendioxid und elektrolytisch erzeugtem Wasserstoff Methanol unter Volllast mit einer Rate von circa 220 t Methanol pro Tag produziert, hat einen Wärmeimportbedarf von mindestens circa 10 MW bei mindestens circa 130 °C.

Gemäß dem folgenden Beispiel überträgt der Elektrolyseur seine Abwärme unmittelbar mittels eines Wärmeübertragers auf das Wärmepumpenmedium des Wärmepumpensystems, und das verdichtete und erwärmte Wärmepumpenmedium überträgt seine Wärme unmittelbar mittels eines Wärmeübertragers auf flüssiges Rohmethanol im Destillationsverdampfer.

Die Abwärme des Elektrolyseurs kann dabei auf folgende Weise übertragen werden:
- 10 MW Abwärme aus dem Prozessmedium des Elektrolyseurs (Elektrolysemedium bei 60 °C) werden auf das Wärmepumpenmedium übertragen, wobei letzteres dadurch verdampft wird und nach Verdampfung eine Temperatur von 50 °C aufweist.
- Das verdampfte Wärmepumpenmedium wird einem Kompressor zugeführt, welcher die Temperatur des Wärmepumpenmediums auf 130 °C erhöht und den Druck des Wärmepumpenmediums erhöht. Unter Berücksichtigung bekannter Hochtemperatur-Wärmepumpenkurven werden für die Verdichtung des 10 MW enthaltenden Wärmepumpenmediums ca. 3,3 MW Leistung (beispielsweise in Form von Elektrizität) benötigt. Die Leistungszahl beträgt somit 3.
- Das 130 °C warme Wärmepumpenmedium kondensiert auf der heißen Seite der Methanol-Destillationskolonne und liefert durch die Kondensationswärme 10 MW im Wärmeübertrager des Destillationsverdampfers.

Zum Vergleich: Soll bei 2,7 bar aus Wasser durch Zufuhr von elektrischer Energie Sattdampf bei 130 °C erzeugt werden, würde dies die gesamten 10 MW verbrauchen, die im Wärmeübertrager des Destillationsverdampfers übertragen werden.

Die Einsparungen durch die Anwendung der vorliegenden Erfindung betragen in diesem Fall 10 MW (voller Stromverbrauch für die Heizung zur Erzeugung von Sattdampf) minus 3,3 MW (Stromverbrauch des Wärmepumpensystems), was 6,7 MW Ersparnis ergibt. Diese Leistung wird "eingespart", was bedeutet, dass diese nicht in Form von zusätzlichem Strom verbraucht wird, sondern durch die Nutzung der Abwärme des Elektrolyseurs zur Verfügung gestellt werden kann.

### Bezugszeichenliste

- 1, 2, 3: Verfahren
- 9: Wärmepumpensystem
- 10: Elektrolyseur
- 11: Wasserreservoir
- 12: Gleichstromquelle
- 13: Methanolsynthese-Reaktoranordnung
- 14: Kohlendioxidquelle
- 15: Destillationskolonne
- 16: Wärmeübertrager (Verdampfer)
- 17: Wärmeübertrager (Verflüssiger)
- 18: Kompressor
- 19: Drosselventil
- 20: Wasserstrom
- 21: Heißes Elektrolysemedium
- 22: Gekühltes Elektrolysemedium
- 23: Wasserstoffstrom
- 24: Kohlendioxidstrom
- 25a: Methanolhaltiger Zulaufstrom
- 25b, 25c, 25d, 25e: Methanolhaltiger Strom
- 26: Methanolstrom
- 27a: Dampfstrom
- 27b: Kondensatstrom
- 28a, 28b, 28c, 28d: Zirkulierendes Wärmepumpenmedium
- 29a, 29b: Strom des ersten Hilfsmediums
- 30a, 30b: Strom des zweiten Hilfsmediums
- 31: Wärmeübertrager erstes Hilfsmedium
- 32: Wärmeübertrager zweites Hilfsmedium
- 33: Treibmittelpumpe
- 34: Saugstutzen
- 35: Druckstutzen
- 36: Treibmedienanschluss
- 37: Dampfstrom
- 38: Kondensatstrom
- 39: Wärmeübertrager

## Patentansprüche

1. Verfahren zur Herstellung von Methanol, umfassend die Verfahrensschritte
a) Bereitstellen eines Synthesegasstroms, welcher zumindest eine Wasserstoffkomponente (23) und eine Kohlenstoffoxidkomponente (24) umfasst, wobei zumindest eine der Komponenten zumindest teilweise durch einen Elektrolyseur (10) bereitgestellt wird;
b) Umsetzen des Synthesegasstroms an einem Methanolsynthesekatalysator in einer Reaktoranordnung (13) zu einem gasförmigen Rohmethanolstrom;
c) Erzeugen eines flüssigen Rohmethanolstroms durch Kondensieren und Abscheiden des gemäß Schritt b) erhaltenen Rohmethanolstroms;
d) Thermisches Abtrennen von Methanol aus dem flüssigen Rohmethanolstrom in einer Destillationsvorrichtung, wobei die Destillationsvorrichtung zumindest eine Destillationskolonne (15) aufweist, in welcher ein methanolhaltiger Strom (25b) in einem Destillationsverdampfer (17, 31) verdampft wird,
**dadurch gekennzeichnet, dass**
in dem Elektrolyseur (10) erzeugte Abwärme durch indirekte Wärmeübertragung auf ein zirkulierendes Wärmepumpenmedium (28a, 28b, 28c, 28d) eines Wärmepumpensystems (9) übertragen wird, wodurch das Wärmepumpenmedium zumindest teilweise verdampft, das Wärmepumpenmedium anschließend verdichtet wird, und das Wärmepumpenmedium durch zumindest teilweises Kondensieren abgegebene Wärme durch indirekte Wärmeübertragung auf ein flüssiges Medium überträgt, und die von dem Wärmepumpensystem auf das flüssige Medium übertragene Wärme zumindest teilweise für die Verdampfung des methanolhaltigen Stroms (25b) im Destillationsverdampfer gemäß Schritt d) genutzt wird.

2. Verfahren nach Anspruch 1, **dadurch gekennzeichnet, dass** von dem Wärmepumpenmedium abgegebene Wärme im Destillationsverdampfer (17, 31) unmittelbar durch indirekte Wärmeübertragung auf den methanolhaltigen Strom (25b) übertragen wird, wobei der methanolhaltige Strom (25b) das flüssige Medium umfasst.

3. Verfahren nach Anspruch 1, **dadurch gekennzeichnet, dass** von dem Wärmepumpenmedium abgegebene Wärme durch indirekte Wärmeübertragung auf ein erstes Hilfsmedium (29a) übertragen wird, wobei das erste Hilfsmedium (29a, 29b) das flüssige Medium umfasst, und von dem ersten Hilfsmedium (29b) aufgenommene Wärme durch indirekte Wärmeübertragung im Destillationsverdampfer (17, 31) auf den methanolhaltigen Strom (25b) übertragen wird.

4. Verfahren nach Anspruch 3, **dadurch gekennzeichnet, dass** die Reaktoranordnung (13) einen wassergekühlten Reaktor beinhaltet, und in einem Kühlsystem des wassergekühlten Reaktors ein Dampfstrom (37) erzeugt wird, und der Dampfstrom (37) zumindest teilweise mit dem ersten Hilfsmedium (29b) zusammengeführt wird, bevor von dem ersten Hilfsmedium (29b) aufgenommene Wärme durch indirekte Wärmübertragung im Destillationsverdampfer (17, 31) auf den methanolhaltigen Strom (25b) übertragen wird.

5. Verfahren nach einem der vorherigen Ansprüche, **dadurch gekennzeichnet, dass** in dem Elektrolyseur (10) erzeugte Abwärme unmittelbar durch indirekte Wärmeübertragung von einem Elektrolysemedium (21) auf das Wärmepumpenmedium (28a) übertragen wird.

6. Verfahren nach einem der Ansprüche 1 bis 4, **dadurch gekennzeichnet, dass** in dem Elektrolyseur (10) erzeugte Abwärme durch indirekte Wärmeübertragung auf ein zweites Hilfsmedium (30b) übertragen wird, und von dem zweiten Hilfsmedium (30a) aufgenommene Wärme auf das Wärmepumpenmedium (28a) übertragen wird.

7. Verfahren nach Anspruch 6, **dadurch gekennzeichnet, dass** die Reaktoranordnung einen wassergekühlten Reaktor beinhaltet, und in einem Kühlsystem des wassergekühlten Reaktors ein Dampfstrom (37) erzeugt wird, und der Dampfstrom (37) zumindest teilweise mit dem zweiten Hilfsmedium (29b) zusammengeführt wird, bevor von dem zweiten Hilfsmedium (29b) aufgenommene Wärme auf das Wärmepumpenmedium übertragen wird.

8. Verfahren nach einem der vorherigen Ansprüche, **dadurch gekennzeichnet, dass** das Wärmepumpenmedium (28a, 28b, 28c, 28d) Wasser beinhaltet, wobei für das Verdichten des Wärmepumpenmediums ein Dampfstrom (37) genutzt wird.

9. Verfahren nach Anspruch 8, **dadurch gekennzeichnet, dass** die Reaktoranordnung (13) einen wassergekühlten Reaktor beinhaltet, und in einem Kühlsystem des wassergekühlten Reaktors ein Dampfstrom (37) erzeugt wird, welcher zumindest teilweise für das Verdichten des Wärmepumpenmediums (28b) genutzt wird.

10. Verfahren nach einem der Ansprüche 8 oder 9, **dadurch gekennzeichnet, dass** das Wärmepumpensystem (9) zum Verdichten des Wärmepumpenmediums (28b) eine Treibmittelpumpe (33) beinhaltet, wobei die Treibmittelpumpe einen Saugstutzen (34), einen Druckstutzen (35), und einen Treibmedienanschluss (36) aufweist, und wobei der Treibmittelpumpe (33) über den Saugstutzen (34) das zu verdichtende Wärmepumpenmedium (28b) zugeführt wird, über den Treibmedienanschluss (36) der Dampfstrom (37) zugeführt wird, und das verdichtete Wärmepumpenmedium (28c) über den Druckstutzen (35) aus der Treibmittelpumpe (33) ausgeleitet wird.

11. Verfahren nach einem der vorherigen Ansprüche, **dadurch gekennzeichnet, dass** das Wärmepumpensystem (9) als Hochtemperaturwärmepumpensystem ausgebildet ist, in welchem das verdichtete Wärmepumpenmedium (28c) eine Temperatur von mindestens 60 °C aufweist, bevorzugt eine Temperatur von mindestens 100 °C aufweist.

12. Verfahren nach einem der vorherigen Ansprüche, **dadurch gekennzeichnet, dass** die Kohlenstoffoxidkomponente (24) des Synthesegasstroms zumindest 50 mol-% Kohlendioxid umfasst, und zumindest ein Teil der Kohlenstoffoxidkomponente durch ein Kohlenstoffdioxid-Abscheideverfahren erzeugt wird.
